(19) Europäisches Patentamt
European Patent Office
Office européen des brevets

(11) Veröffentlichungsnummer: **0 113 093**
**B1**

# (12) EUROPÄISCHE PATENTSCHRIFT

(45) Veröffentlichungstag der Patentschrift: **21.05.86**

(51) Int. Cl.⁴: **C 07 D 215/56**, A 61 K 31/495

(21) Anmeldenummer: **83112725.3**

(22) Anmeldetag: **17.12.83**

(54) **1-Cyclopropyl-6-fluor-1,4-dihydro-4-oxo-7-(alkyl-1-piperazinyl)-3-chinolincarbonsäuren, Verfahren zu ihrer Herstellung sowie diese enthaltende antibakterielle Mittel.**

(30) Priorität: **29.12.82 DE 3248506**

(43) Veröffentlichungstag der Anmeldung: **11.07.84 Patentblatt 84/28**

(45) Bekanntmachung des Hinweises auf die Patenterteilung: **21.05.86 Patentblatt 86/21**

(84) Benannte Vertragsstaaten: **AT BE CH DE FR GB IT LI LU NL SE**

(56) Entgegenhaltungen:
**EP - A - 0 004 279**
**EP - A - 0 049 355**
**EP - A - 0 078 362**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Petersen, Uwe, Dr., Auf dem Forst 4, D-5090 Leverkusen 1 (DE)**
Erfinder: **Grohe, Klaus, Dr., Am Wasserturm 10, D-5068 Odenthal (DE)**
Erfinder: **Zeiler, Hans-Joachim, Dr., Elsbeekerstrasse 46, D-5620 Velbert 15 (DE)**
Erfinder: **Metzger, Karl Georg, Dr., Pahlkestrasse 15, D-5600 Wuppertal 1 (DE)**

EP 0 113 093 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue 1-Cyclopropyl-6-fluor-1,4-dihydro-4-oxo-7-(alkyl-1-piperazinyl)-3-chinolincarbonsäuren, Verfahren zu ihrer Herstellung sowie diese enthaltende antibakterielle Mittel und Futterzusatzmittel.

Es ist bereits bekannt geworden, daß 1-Ethyl-6-fluor-1,4-dihydro-4-oxo-7-(1-piperazinyl)-3-chinolincarbonsäure [Norfloxacin) antibakterielle Eigenschaften besitzt (J.Med.Chem. 23, 1358 (1980)].

Es wurde nun gefunden, daß die neuen 1-Cyclopropyl-6-fluor-1,4-dihydro-4-oxo-7-(alkyl-1-piperazinyl)-3-chinolincarbonsäuren der Formel (I)

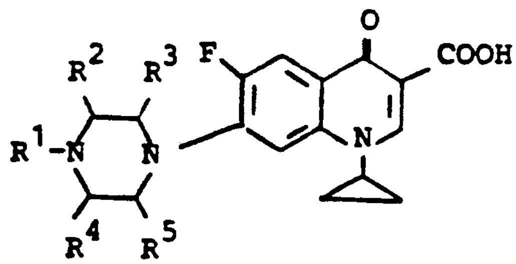

(I)

in der

$R^1$ für Wasserstoff oder gegebenenfalls durch Hydroxy substituiertes Alkyl mit 1 bis 12, vorzugsweise 1 bis 4, Kohlenstoffatomen steht,

$R^2$, $R^3$, $R^4$ und $R^5$ gleich oder verschieden sein können und für Wasserstoff oder Alkyl mit 1 bis 4, vorzugsweise 1 bis 2, Kohlenstoffatomen stehen, wobei mindestens einer der Reste $R^2$ bis $R^5$ Alkyl bedeutet, und deren pharmazeutisch verwendbaren Säureadditions-, Alkali- und Erdalkalisalze und Hydrate eine hohe antibakterielle Wirkung aufweisen.

Weiterhin wurde gefunden, daß man die 1-Cyclopropyl-6-fluor-1,4-dihydro-4-oxo-7-(alkyl-1-piperazinyl)-3-chinolincarbonsäuren der Formel (I) erhält, wenn man 7-Chlor-1-cyclopropyl-6-fluor-1,4-dihydro-4-oxo-3-chinolincarbonsäure der Formel (II)

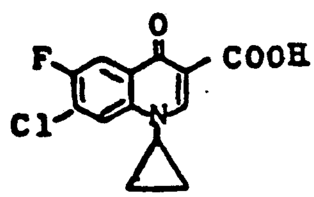

(II)

mit Piperazinderivaten der Formel (III)

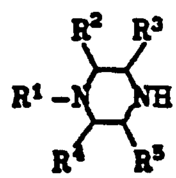

(III)

in welcher

$R^1$,$R^2$,$R^3$,$R^4$ und R die oben angegebenen Bedeutungen haben,

gegebenenfalls in Gegenwart von Säurebindern umsetzt (Methode A).

Die erfindungsgemäßen Verbindungen können auch erhalten werden, indem man Verbindungen der Formel (IA) (= I, $R^1$ = H)

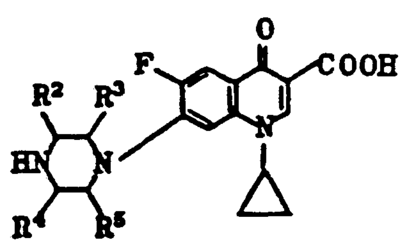

(IA)

in welcher

$R^2$,$R^3$,$R^4$ und $R^5$ die oben angegebene Bedeutung haben,

mit Verbindungen der Formel (IV)

$R^1$-X (IV)

in welcher

$R^1$ die oben angegebene Bedeutung hat, jedoch nicht Wasserstoff sein kann, und

X Chlor, Brom oder Iod bedeutet,

in Gegenwart von Säurebindern umsetzt (Methode B).

Überraschenderweise zeigen die erfindungsgemäßen Verbindungen nicht nur eine höhere antibakterielle Wirksamkeit als die aus dem Stand der Technik bekannten Verbindungen, sondern auch eine deutlich verbesserte Resorbierbarkeit. Sie eignen sich daher als Wirkstoffe für die Human- und Veterinärmedizin, wobei zur Veterinärmedizin auch die Vorbeugung und Behandlung bei Fischen zu zählen ist. Die erfindungsgemäßen Stoffe stellen somit eine Bereicherung der Pharmazie dar.

Verwendet man bei der Umsetzung nach der Methode A 2-Methylpiperazin und 7-Chlor-1-cyclopropyl-6-fluor-1,4-dihydro-4-oxo-3-chinolincarbonsäure als Ausgangsstoffe, so kann der Reaktionsablauf durch das folgende Formelschema wiedergegeben werden:

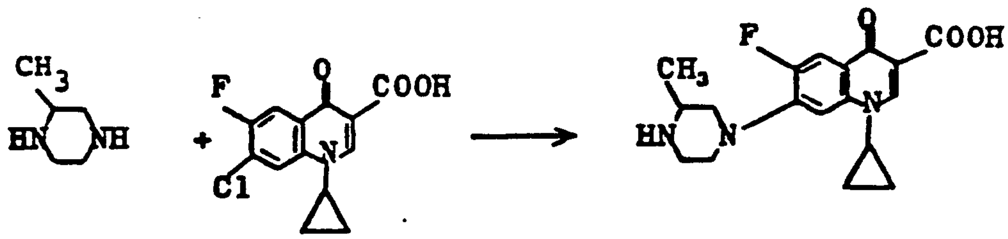

Verwendet man bei der Umsetzung nach der Methode B Ethyliodid und 1-Cyclopropyl-6-fluor-1,4-dihydro-4-oxo-7-(3-methyl-1-piperazinyl)-3-chinolincarbonsäure als Ausgangsstoffe, so kann der Reaktionsablauf durch das folgende Formelschema wiedergegeben werden:

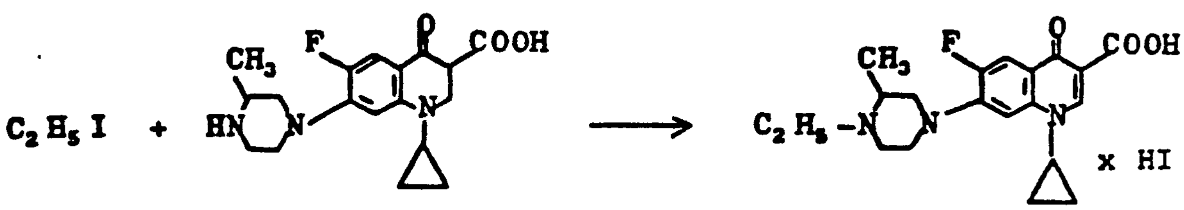

Die als Ausgangsstoff nach Methode A verwendbare 7-Chlor-1-cyclopropyl-6-fluor-1,4-dihydro-4-oxo-3-chinolincarbonsäure der Formel (II) kann gemäß folgendem Reaktionsschema hergestellt werden:

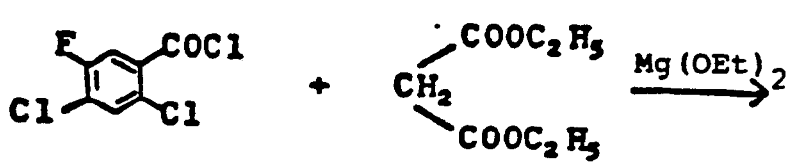


(1) (2)

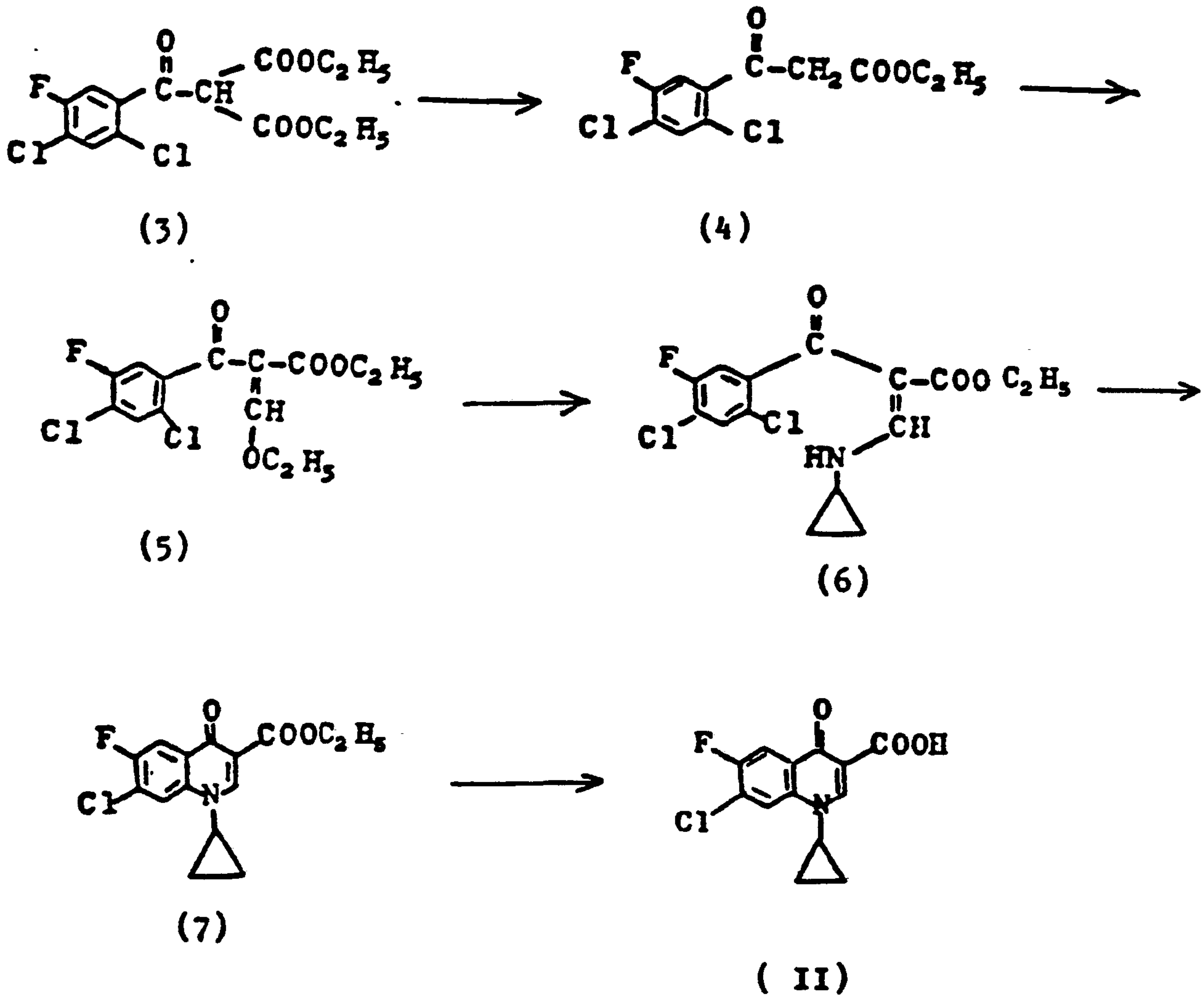

(3) (4) (5) (6) (7) (II)

Danach wird Malonsäurediethylester (2) mit (1) in Gegenwart von Magnesiumalkoholat mit 2,4-Dichlor-5-fluor-benzoylchlorid (Deutsche Patentanmeldung Nr. 3142856.8) zum Acylmalonester (3) acyliert (Organikum, 3. Aufl. 1964, S. 438).

Durch partielle Verseifung und Decarboxylierung von (3) in wäßrigem Medium mit katalytischen Mengen Schwefelsäure oder p-Toluolsulfonsäure erhält man in guter Ausbeute den Aroylessigsäureethylester (4), der mit o-Ameisensäuretriethylester/Acetanhydrid in den 2-(2,4-Dichlor-5-fluor-benzoyl)-3-ethoxy-acrylsäureethylester (5) übergeht. Die Umsetzung von (5) mit Cyclopropylamin in einem Lösungsmittel, wie z.B. Methylenchlorid, Alkohol, Chloroform, Cyclohexan oder Toluol führt in leicht exothermer Reaktion zum gewünschten Zwischenprodukt (6).

Die Cyclisierungsreaktion (6) → (7) wird in einem Temperaturbereich von etwa 60 bis 300°C, bevorzugt 80 bis 180°C durchgeführt.

Als Verdünnungsmittel können Dioxan, Dimethylsulfoxid, N-Methyl-pyrrolidon, Sulfolan, Hexamethylphosphorsäuretriamid und bevorzugt N,N-Dimethylformamid verwendet werden.

Als Säurebinder kommen für diese Reaktionsstufe Kaliumtert.-butanolat, Butyl-lithium, Lithium-phenyl, Phenylmagnesiumbromid, Natriummethylat, Natriumhydrid und besonders bevorzugt Kalium- oder Natriumcarbonat in Betracht. Es kann vorteilhaft sein, einen Überschuß von 10 Mol-8 an Base einzusetzen.

Die in dem letzten Schritt erfolgende Esterhydrolyse unter basischen oder sauren Bedingungen führt zur 7-Chlor-1-cyclopropyl-6-fluor-1,4-dihydro-4-oxo-3-chino-lincarbonsäure (II).

Das als Ausgangsmaterial für diesen Syntheseweg verwendete 2,4-Dichlor-5-fluor-benzoylchlorid (1) und die entsprechende Carbonsäure sowie das für die Herstellung von (1) benötigte 3-Fluor-4,6-dichlortoluol (10) sind noch nicht bekannt.

Das nachstehende Formelschema zeigt die Herstellung dieser Vor- bzw. Zwischenprodukte, ausgehend von 2,4-Dichlor-5-methyl-anilin (8).

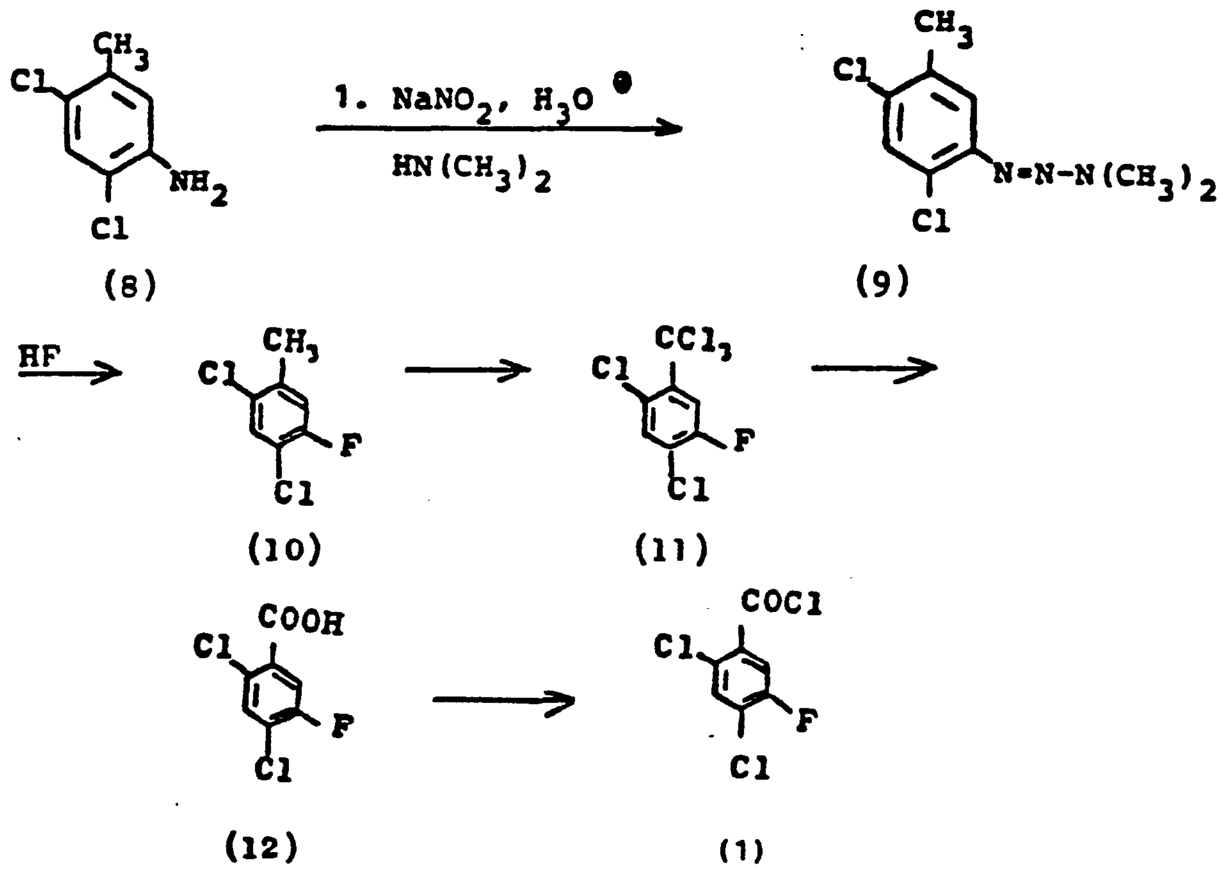


(8) (9) (10) (11) (12) (1)

Danach wird 2,4-Dichlor-5-methyl-anilin (8) mit Hilfe von $NaNO_2$ diazotiert und das dabei entstehende Diazoniumsalz mit Dimethylamin in das Triazen (9) übergeführt.

Das Triazen (9) wird in überschüssiger wasserfreier HF gelöst. Dabei spaltet sich das Triazen in 2,4-Dichlor-5-methylbenzol-diazoniumfluorid und Dimethylamin. Ohne Zwischenisolierung wird diese Lösung thermisch bei 130 bis 140°C unter $N_2$-Abspaltung in 3-Fluor-4,6-dichlor-toluol (10) gespalten. Ausbeute: 77,7 % der Theorie.

Das 3-Fluor-4,6-dichlortoluol (10) wird in einem Temperaturbereich von 110 bis 160°C unter UV-Bestrahlung zu 2,4-Dichlor-5-fluor-1-trichlormethylbenzol (11) chloriert.

Die Verseifung von (11) mit 95 %iger Schwefelsäure führt zu 2,4-Dichlor-5-fluor-benzoesäure (12), die mit Thionylchlorid in das Carbonsäurechlorid (1) (Sdp. 121°C/ 20 mbar; $n_D^{20}$ 1,5722) übergeht.

Die als Ausgangsstoffe verwendeten Piperazinderivat sind bekannt oder können nach literaturbekannten Verfahren erhalten werden. Als Beispiele seien genannt:

2-Methylpiperazin, 1,2-Dimethylpiperazin, cis- und trans-2,5-Dimethylpiperazin, cis- und trans-2,6-Dimethylpiperazin, 2,3-Dimethylpiperazin, 2,3,5-Trimethylpiperazin, 2,3,5,6-Tetramethylpiperazin, 2-Ethylpiperazin, 2-Ethyl-3,6-dimethylpiperazin, 2-Propylpiperazin, 2-Isopropyl-piperazin, 2-Isobutylpdperazin.

Die als Ausgangsstoffe verwendeten Alkylhalogenide (IV) sind bekannt. Als Beispiele seien genannt: Methyliodid, Methylbromid, Ethyliodid, Ethylbromid, Ethylchlorid, 2-Hydroxyethylchlorid, 3-Hydroxypropylchlorid, 4-Rydroxybutylchlorid, n-Propylbromid, i-Propyliodid, n-Butylbromid, i-Butylbromid, sek.-Butylchlorid, n-Pentylchlorid, 3-Methylbutylchlorid, n-Dodecylchlorid.

Die Umsetzung von (II) mit (III) gemäß Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo[2,2,2]octan (DABCO), überschüssiges Piperazin-Derivat (III) oder 1,8-Diazabicyclo[5,4,0]undec-7-en (DBU).

Die Reaktionstemperaturen können in einem größeren Bereich variiert werden. Im allgemeinen arbeitet man zwischen etwa 20 und 200° C, vorzugsweise zwischen 80 und 180° C.

Die Umsetzung kann bei Normaldruck, aber auch bei erhöhtem Druck, durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure (II) 1 bis 15 Mol, vorzugsweise 1 bis 6 Mol, des Piperazin-Derivats(III) ein.

Die Umsetzung von (IA) mit (IV) wird vorzugsweise in einem Verdünnungsmittel wie Dimethylsulfoxid, Dioxan, N,N-Dimethylformamid, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2,2,2]octan (DABCO) oder 1,8-Diazabicyclo-[5,4,0]undec-7-en (DBU).

Die Reaktionstemperaturen können in einem größeren Bereich variiert werden. Im allgemeinen arbeitet man zwischen etwa 20 und etwa 180° C, vorzugsweise zwischen 40 und 110° C.

Bei der Durchführung des erfindungsgemäßen Verfahrens gemäß Methode B setzt man auf 1 Mol der Verbindung (IA) 1 bis 4 Mol, vorzugsweise 1 bis 1,5 Mol, des Alkylierungsmittels (IV)ein.

Als neue Wirkstoffe seien im einzelnen genannt:

1-Cyclopropyl-6-fluor-1,4-dihydro-4-oxo-7-(3-methyl-1-piperazinyl)-3-chinolincarbonsäure,
1-Cyclopropyl-6-fluor-1,4-dihydro-4-oxo-7-(3,4-dimethyl-1-piperazinyl)-3-chinolincarbonsäure,
1-Cyclopropyl-6-fluor-1,4-dihydro-4-oxo-7-(4-ethyl-3-methyl-1-piperazinyl)-3-chinolincarbonsäure,
1-Cyclopropyl-6-fluor-1,4-dihydro-4-oxo-7-[4-(2-hydroxy-ethyl)-3-methyl-1-piperazinyl]-3-chinolincarbonsäure,
1-Cyclopropyl-6-fluor-1,4-dihydro-4-oxo-7-[4-(3-hydroxy-propyl)-3-methyl-1-piperazinyl]-3-chinolincarbonsäure,
1-Cyclopropyl-6-fluor-1,4-dihydro-4-oxo-7-(2,5-dimethyl-1-piperazinyl)-3-chinolincarbonsäure,
1-Cyclopropyl-6-fluor-1,4-dihydro-4-oxo-7-(2,4,5-tri-methyl-1-piperazinyl)-3-chinolincarbonsäure,
1-Cyclopropyl-6-fluor-1,4-dihydro-4-oxo-7-(4-ethyl-2,5-dimethyl-1-piperazinyl)-3-chinolincarbonsäure,
1-Cyclopropyl-6-fluor-1,4-dihydro-4-oxo-7-(3,5-dimethyl-1-piperazinyl)-3-chinolincarbonsäure,
1-Cyclopropyl-6-fluor-1,4-dihydro-4-oxo-7-(3,4,5-trime-thyl-1-piperazinyl)-3-chinolincarbonsäure,
1-Cyclopropyl-6-fluor-1,4-dihydro-4-oxo-7-(4-ethyl-3,5-dimethyl-1-piperazinyl)-3-chinolincarbonsäure,
1-Cyclopropyl-6-fluor-1,4-dihydro-4-oxo-7-(3-ethyl-1-piperazinyl)-3-chinolincarbonsäure,
1-Cyclopropyl-6-fluor-1,4-dihydro-4-oxo-7-(3-n-propyl-1-piperazinyl)-3-chinolincarbonsäure,
1-Cyclopropyl-6-fluor-1,4-dihydro-4-oxo-7-(3-isopropyl-1-piperazinyl)-3-chinolincarbonsäure,
1-Cyclopropyl-6-fluor-1,4-dihydro-4-oxo-7-(3-isobutyl-1-piperazinyl)-3-chinolincarbonsäure,
1-Cyclopropyl-6-fluor-1,4-dihydro-4-oxo-7-(3-methyl-4-n-propyl-1-piperazinyl)-3-chinolincarbonsäure,
1-Cyclopropyl-6-fluor-1,4-dihydro-4-oxo-7-(3-methyl-4-isopropyl-1-piperazinyl)-3-chinolincarbonsäure,
1-Cyclopropyl-6-fluor-1,4-dihydro-4-oxo-7-(4-n-butyl-3-methyl-1-piperazinyl)-3-chinolincarbonsäure,
1-Cyclopropyl-6-fluor-1,4-dihydro-4-oxo-7-(4-isobutyl-3-methyl-1-piperazinyl)-3-chinolincarbonsäure,
1-Cyclopropyl-6-fluor-1,4-dihydro-4-oxo-7-(4-sek.-butyl-3-methyl-1-piperazinyl)-3-chinolincarbonsäure,
1-Cyclopropyl-6-fluor-1,4-dihydro-4-oxo-7-(4-tert.-butyl-3-methyl-1-piperazinyl)-3-chinolincarbonsäure,
1-Cyclopropyl-6-fluor-1,4-dihydro-4-oxo-7-(3-methyl-4-n-pentyl-1-piperazinyl)-3-chinolincarbonsäure,
1-Cyclopropyl-6-fluor-1,4-dihydro-4-oxo-7-(4-n-dodecyl-3-methyl-1-piperazinyl)-3-chinolincarbonsäure
und deren pharmazeutisch verwendbaren Säureadditionssalze, Alkalisalze, Erdalkalisalze oder Hydrate.

Die erfindungsgemäßen Verbindungen zeigen ein breites antibakterielles Spektrum gegen gram-positive und gramnegative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracyline.

Die erfindungsgemäßen Verbindungen weisen bei geringer Toxizität eine starke und breite antimikrobielle Wirksamkeit auf. Diese Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. Epidermidis, (Staph. = Staphylococcus); Lactobacteriaceae, wie Streptokokken, z.B. Streptococcus pyogenes, α- bzw. ß-hämolysierende Streptokokken, nicht (γ-)hämolysierende Streptokokken, Enterokokken und Diplococcus pneumoniae (Pneumokokken) (Str. = Streptococcus); Enterobacteriaceae, wie Escherichiae-Bakterien der Coli-Gruppe: Escherichia-Bakterien, z.B. Escherichia coli, Enterobacter-Bakterien, z.B. aerogenes, E. Cloacae, Rlebsiella-Bakterien, z.B. K. pneumoniae, Serratia z.B. Serratia marcescens (E. = Enterobacter) (K. = Klebsiella), Proteae-Bakterien der Proteus-Gruppe: Proteus, z.B. Proteus vulgaris, Pr.morganii, Pr.rettgeri, Pr.mirabilis (Pr. = Proteus);

Pseudomonadaceae, wie Pseudomonas-Bakterien, z.B. Pseudomonas aeruginosa (PS. = Pseudomonas);

Bacteroidaceae, wie Bacteroides-Bakterien, z.B. Bacteroides fragilis (B. = Bacteroides); Mykoplasmen, z.B. Mykoplasma pneumoniae.

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können, seien beispielsweise genannt:

Erkrankungen der Atmungswege und des Rachenraumes:

Otitis; Pharyngitis; Pneumonie; Peritonitis; Pyelonephritis; Cystitis; Endocarditis; Systeminfektionen; Bronchitis; Arthritis; lokale Infektionen, septische Erkrankungen.

Zur Erfindung gehören pharmazeutische Zubereitungen, die neben nicht-toxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen,; sowie Verfahren zur Herstellung dieser Zubereitungen.

Zur Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Raolin und Bentonit und (i) Gleitmittel, z.B. Talkum, Calcium- und Magnesiumstearat und feste. Polyethylenglykole oder Gemische der unter (a) bis (i) aufgeführten Stoffe.

Die Tabletten, Dragees, Rapseln, Pillen und Granulate können mit den üblichen, gegebenenfalls Opakisierungsmittel enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser

Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Bu-tylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Ronservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die folgenden Herstellungsbeispiele erläutern die Erfindung:

**Beispiel A** (Herstellung des Ausgangsproduktes II):

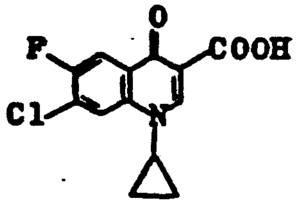

24,3 g Magnesiumspäne werden in 50 ml wasserfreiem Ethanol suspendiert. Man versetzt mit 5 ml Tetrachlorkohlenstoff und tropft, wenn die Reaktion in Gang gekommen ist, ein Gemisch von 160 g Malonsäurediethylester, 100 ml abs. Ethanol und 400 ml wasserfreiem Ether zu, wobei ein heftiger Rückfluß zu beobachten ist. Nach dem Abklingen der Reaktion wird noch 2 Stunden zum Sieden erhitzt, mit Trockeneis/Aceton auf −5°C bis −10°C gekühlt und bei dieser Temperatur eine Lösung von 227,5 g 2,4-Dichlor-5-fluor-benzoylchlorid (1) in 100 ml abs. Ether langsam zugetropft. Man rührt 1 Stunde bei 0 bis -5°C, läßt über Nacht auf Raumtemperatur kommen und läßt unter Eiskühlung ein Gemisch von 400 ml Eiswasser und 25 ml konz. Schwefelsäure zulaufen. Die Phasen werden getrennt und zweimal mit Ether nachextrahiert. Die vereinigten Etherlösungen werden mit gesättigter NaCl-Lösung gewaschen, mit $Na_2SO_4$ getrocknet und das Lösungsmittel im Vakuum abgezogen. Man erhält 349,5 g 2,4-Dichlor-5-fluor-benzoyl-malonsäure-diethylester (3) als Rohprodukt.

Eine Emulsion von 34,9 g rohem 2,4-Dichlor-5-fluor-benzoyl-malonsäurediethylester (3) in 50 ml Wasser wird

mit 0,15 g p-Toluolsulfonsäure versetzt. Man erhitzt unter gutem Rühren 3 Stunden zum Sieden, extrahiert die erkaltete Emulsion mehrmalsmit Methylenchlorid, wäscht die vereinigten $CH_2Cl_2$-Lösungen einmal mit gesättigter NaCl-Lösung, trocknet mit $Na_2SO_4$ und destilliert das Lösungsmittel im Vakuum ab. Die Fraktionierung des Rückstandes im Feinvakuum liefert 21,8 g 2,4-Dichlor-5-fluor-benzoyl-essigsäureethylester (4) vom Siedepunkt 127-142° C/0,09 mbar.

Ein Gemisch von 21,1 g 2,4-Dichlor-5-fluor-benzoyl-essigsäureethylester (4), 16,65 g o-Ameisensäureethylester und 18,55 g Acetanhydrid wird 2 Stunden auf 150° C erhitzt. Dann werden im Wasserstrahlvakuum und zuletzt im Feinvakuum bei einer Badtemperatur von 120° C die flüchtigen Bestandteile abdestilliert. Zurück bleiben 25,2 g roher 2-(2,4-Dichlor-5-fluor-benzoyl)-3-ethoxy-acrylsäureethylester (5). Er ist genügend rein für die weiteren Umsetzungen.

Eine Lösung von 24,9 g 2-(2,4-Dichlor-5-fluor-benzoyl)-3-ethoxy-acrylsäureethylester (5) in 80 ml Ethanol wird unter Eiskühlung und Rühren tropfenweise mit 4,3 g Cyclopropylamin versetzt. Wenn die exotherme Reaktion abgeklungen ist, wird noch 1 Stunde bei Raumtemperatur gerührt, das Lösungsmittel im Vakuum abgezogen und der Rückstand aus Cyclohexan/Petrolether umkristallisiert. Man erhält 22,9 g 2-(2,4-Dichlor-5-fluor-benzoyl)-3-cyclopropylamino-acrylsäureethylester (6) vom Schmelzpunkt 89-90° C.

Eine Lösung von 31,9 g 2-(2,4-Dichlor-5-fluor-benzoyl)-3-cyclopropylamino-acrylsäure-ethylester (6) in 100 ml wasserfreiem Dioxan wird unter Eiskühlung und Rühren portionsweise mit 3,44 g 80 %igem Natriumhydrid versetzt. Dann wird 30 Minuten bei Raumtemperatur und 2 Stunden unter Rückfluß gerührt und das Dioxan im Vakuum abgezogen. Der Rückstand (40,3 g) wird in 150 ml Wasser suspendiert, mit 6,65 g Ätzkali versetzt und 1,5 h refluxiert. Man filtriert die warme Lösung und wäscht mit $H_2O$ nach. Dann wird mit halbkonzentrierter Salzsäure unter Eiskühlung auf pH = 1 bis 2 angesäuert, der Niederschlag abgesaugt, mit Wasser gewaschen und im Vakuum bei 100° C getrocknet. Man erhält auf diese Weise 27,7 g 7-Chlor-1-cyclopropyl-6-fluor-1,4-dihydro-4-oxo-3-chino-lincarbonsäure (II) vom Schmelzpunkt 234-237° C.

**Beispiel 1**

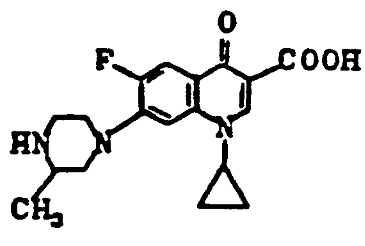

x 1/2 $H_2O$

Ein Gemisch aus 2,8 g (0,01 Mol) 7-Chlor-1-cyclopropyl-6-fluor-1,4-dihydro-4-oxo-3-chinolincarbonsäure (III) und 5 1 g (0 051 Mol) 2-Methylpiperazin in 6 ml Dimethylsulfoxid wird 2 Stunden auf 140° C erhitzt. Anschließend wird das Lösungsmittel im Hochvakuum abdestilliert, der Rückstand mit 6 ml heißem Wasser versetzt und 1 Stunde auf 95° C gehalten. Man kühlt mit Eis, saugt den ausgefallenen Niederschlag ab, wäscht mit etwas Wasser und löst ihn in einer Mischung von 0,8 ml Essigsäure und 10 ml Wasser bei 90 bis 100° C auf. Das Filtrat wird mit Kalilauge (0,75 g KOH in 0,7 ml Wasser) auf pH 8 gebracht und der ausgefallene Niederschlag aus Methanol umkristallisiert. Man erhält 1,8 g (52 % der Theorie) 1-Cyclopropyl-6-fluor-1,4-dihydro-4-oxo-7-(3-methyl-1-piperazinyl)-3-chinolincarbonsäure-semihydrat mit einem Zersetzungspunkt von 230 bis 232° C.

**Beispiel 2**

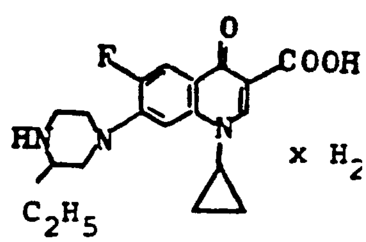

x $H_2O$

Eine Mischung von 2,8 g (0,01 Mol) 7-Chlor-1-cyclo-propyl-6-fluor-1,4-dihydro-4-oxo-3-chinolincarbon-säure (II) und 3,4 g (0,03 Mol) 2-Ethylpiperazin in 15 ml Dimethylsulfoxid wird 2 Stunden auf 140° C erhitzt. Die Lösung wird im Hochvakuum eingeengt, der Rückstand mit 30 ml Wasser auf 90° C erhitzt, der ausgefallene Niederschlag abgesaugt, mit Wasser und Methanol gewaschen und isoliert. Man erhält 1,1 g (31 % der Theorie) 1-Cyclopropyl-6-fluor-1,4-dihydro-4-oxo-7-(3-ethyl-1-piperazinyl)-3-chinolincarbonsäure-monohydrat mit einem Zersetzungspunkt von 255-258° C.

**Beispiel 3**

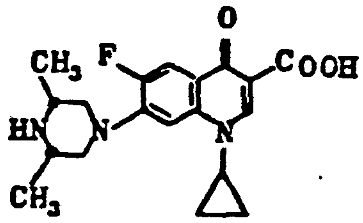

Ein Gemisch aus 2,8 g (0,01 Mol) 7-Chlor-1-cyclopropyl-6-fluor-1,4-dihydro-4-oxo-3-chinolincarbonsäure, 1,14 g (0,01 Mol) cis-2,6-Dimethylpiperazin und 2,2 g Diaza-bicyclo[2,2,2]octan wird 5 Stunden auf 140° C erhitzt. Das Lösungsmittel wird im Hochvakuum abdestilliert, der Rückstand mit 30 ml Wasser versetzt, die Suspension mit 2 n HCl auf pH 8 eingestellt und der ausgefallene Niederschlag mit 30 ml Methanol ausgekocht. Man erhält 0,75 g (21 % der Theorie) 1-Cyclopropyl-6-fluor-1,4-dihydro-4-oxo-7-(3,5-dimethyl-1-piperazinyl)-3-chinolincarbonsäure vom Zersetzungspunkt 234-236° C. Massenspektrum: 359 (M+), 290, 289 (100 %, M+-70), 245, 70.

**Beispiel 4**

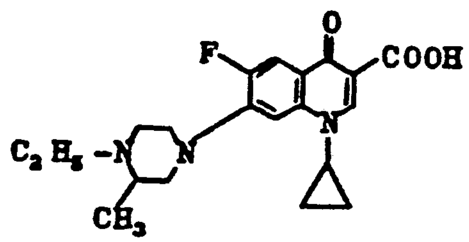

xHIx$H_2O$

Ein Gemisch aus 1,7 g (0,005 Mol) 1-Cyclopropyl-6-fluor-1,4-dihydro-4-oxo-7-(3-methyl-1-piperazinyl)-3-chinolincarbonsäure, 1,7 g (0,011 Mol) Ethyliodid und 1,1 g Triethylamin in 20 ml DMF wird 3 Stunden auf 80° C erhitzt. Die Lösung wird im Vakuum eingeengt, der kristalline Rückstand mit 10 ml Wasser verrührt und das ungelöste Produkt aus Methanol umkristallisiert. Man erhält 0,6 g (32 % der Theorie) 1-Cyclopropyl-6-fluor-1,4-dihydro-4-oxo-7-(4-ethyl-3-methyl-1-piperazinyl)-3-chinolincarbon säure-hydroiodid-monohydrat mit einem Zersetzungspunkt von 285-288° C.

**Beispiel 5**

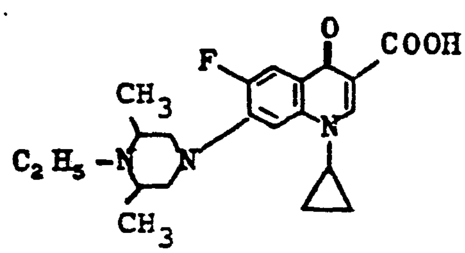

xHI

Man setzt 1,8 g (0,005 Mol) 1-Cyclopropyl-6-fluor-1,4-dihydro-4-oxo-7-(3,5-dimethyl-1-piperazinyl)-3-chinolincarbonsäure analog Beispiel 4 um und isoliert 0,75 g (39 %) 1-Cyclopropyl-6-fluor-1,4-dihydro-4-oxo-7-(4-ethyl-3,5-dimethyl-1-piperazinyl)-3-chinolincarbonsäure-hydroiodid mit einem Zersetzungspunkt von 277-279° C.

Analog Beispiel 4 werden folgende Verbindungen erhalten:

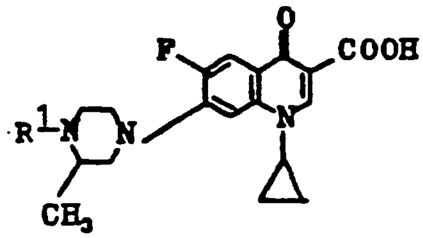

Beispiel $R^1$ Schmp. °C
6 $n\text{-}C_3H_7 \times HBr$ 293-295 (Zers.)
7 $i\text{-}C_3H_7 \times HI$ 289-291 (Zers.)
8 $i\text{-}C_4H_9$ 198-200
9 $n\text{-}C_5H_9 \times HCl$ 238-240 (Zers.)
10 $n\text{-}C_{12}H_{25} \times HCl$ 142-145
11 $HO\text{-}CH_2CH_2CH_2$ 198-201 (Zers.)
12 $HO\text{-}CH_2CH_2 \times HCl$ 240-243(Zers.)

In der nachstehenden Tabelle sind die minimalen Hemmkonzentrationen (MHR) bei verschiedenen Bakterien für einige der erfindungsgemäßen Verbindungen angegeben.

Minimale Hemmkonzentrationen in mcg/ml im Agarverdünnungstest; Denley-Multipoint-Inokulationsverfahren

| Stamm | Beispiel 1 | Beispiel 3 | Beispiel 4 | Beispiel 5 |
|---|---|---|---|---|
| E. coli Neumann | ≤0,015 | ≤0,015 | 0,03 | 0,03 |
| Klebsiella 8085 | ≤0,015 | ≤0,015 | ≤0,015 | 0,03 |
| Klebsiella 6179 | 0,125 | 0,25 | 0,25 | 0,5 |
| Klebsiella 57 USA | 0,03 | 0,125 | 0,25 | 0,5 |
| Providencia 12052 | 16 | 16 | 32 | 32 |
| Serratia 16040 | 4 | 16 | 16 | 32 |
| Staphylococcus Fk422 | 0,25 | 0,5 | 0,5 | 0,5 |
| Staphylococcus 1756 | 0,25 | 0,25 | 0,5 | 0,5 |
| Staphylococcus 133 | 0,25 | 0,25 | 0,25 | 0,5 |

**Patentansprüche**

1) 1-Cyclopropyl-6-fluor-1,4-dihydro-4-oxo-7-(alkyl-1-piperazinyl)-3-chinolincarbonsäuren der Formel (I)

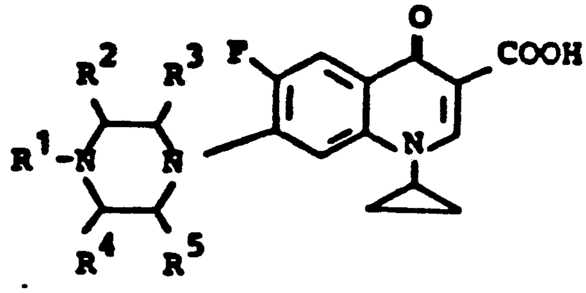

(I)

in der
$R^1$ für Wasserstoff oder gegebenenfalls durch Hydroxy substituiertes Alkyl mit 1 bis 12 Kohlenstoffatomen steht,
$R^2$, $R^3$, $R^4$ und $R^5$ gleich oder verschieden sein können und für Wasserstoff oder Alkyl mit 1 bis 4 Kohlenstoffatomen stehen, wobei mindestens einer der Reste $R^2$ bis $R^5$ Alkyl bedeutet,
und deren pharmazeutisch verwendbare Säureadditions-, Alkali- und Erdalkalisalze und Hydrate.

2) 1-Cyclopropyl-6-fluor-1,4-dihydro-4-oxo-7-(alkyl-1-piperazinyl)-3-chinolincarbonsäuren der Formel (I)

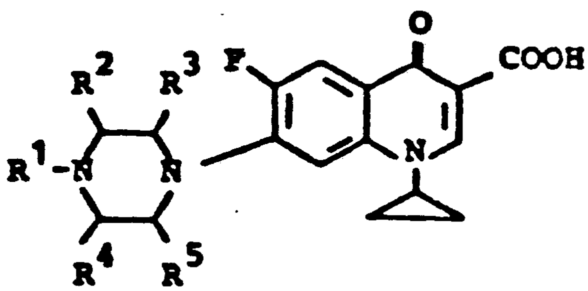

(I)

in der
$R^1$ für Wasserstoff oder gegebenenfalls durch Hydroxy substituiertes Alkyl mit 1 bis 4 Kohlenstoffatomen steht, $R^2$, $R^3$, $R^4$ und $R^5$ gleich oder verschieden sein können und für Wasserstoff oder Alkyl mit 1 bis 2 Kohlenstoffatomen stehen, wobei mindestens einer der Reste $R^2$ bis $R^5$ Alkyl bedeutet, und deren pharmazeutisch verwendbare Säureadditions-, Alkali- und Erdalkalisalze und Hydrate.

3) 1-Cyclopropyl-6-fluor-1,4-dihydro-4-oxo-7-(3-methyl-1-piperazinyl)-3-chinolincarbonsäure.

4) 1-Cyclopropyl-6-fluor-1,4-dihydro-4-oxo-7-(3-ethyl-1-piperazinyl)-3-chinolincarbonsäure.

5) 1-Cyclopropyl-6-fluor-1,4-dihydro-4-oxo-7-(3,5-dimethyl-1-piperazinyl)-3-chinolincarbonsäure.

6) 1-Cyclopropyl-6-fluor-1,4-dihydro-4-oxo-7-(4-ethyl-3-methyl-1-piperazinyl)-3-chinolincarbonsäure.

7) 1-Cyclopropyl-6-fluor-1,4-dihydro-4-oxo-7-(4-ethyl-3,5-dimethyl-1-piperazinyl)-3-chinolincarbonsäure.

8) 1-Cyclopropyl-6-fluor-1,4-dihydro-4-oxo-7-(alkyl-1-piperazinyl)-3-chinolincarbonsäuren der Formel (I)

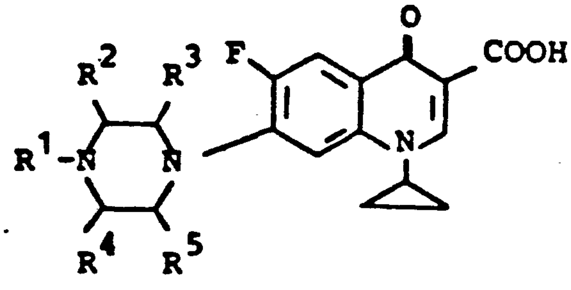

(I)

$R^1$ für Wasserstoff oder gegebenenfalls durch Hydroxy substituiertes Alkyl mit 1 bis 12 Kohlenstoffatomen steht,

$R^2$, $R^3$, $R^4$ und $R^5$ gleich oder verschieden sein können und für Wasserstoff oder Alkyl mit 1 bis 4 Kohlenstoffatomen stehen, wobei mindestens einer der Reste $R^2$ bis $R^5$ Alkyl bedeutet,

und deren pharmazeutisch verwendbare Säureadditions-Alkali- und Erdalkalisalze und Hydrate zur Behandlung bakterieller Erkrankungen.

9) Verfahren zur Herstellung von 1-Cyclopropyl-6-fluor-1,4-dihydro-4-oxo-7-(alkyl-1-piperazinyl)-3-chinolincarbonsäuren der Formel (I) in Anspruch 1, dadurch gekennzeichnet, daß man entweder

a) 7-Chlor-1-cyclopropyl-6-fluor-1,4-dihydro-4-oxo-3-chinolincarbonsäuren der Formel (II)

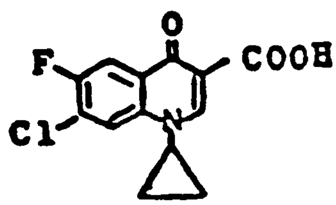

(II)

mit piperazinderivaten der Formel (III)

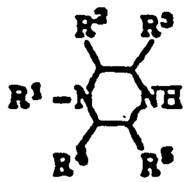

(III)

in welcher

$R^1$, $R^2$, $R^3$, $R^4$ und $R^5$ die oben angegebenen Bedeutungen haben,

in Gegenwart von Säurebindern umsetzt oder

b) Verbindungen der Formel (IA) (= I, $R^1$ = H)

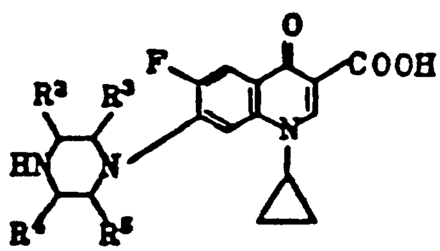

(IA)

in welcher

$R^2$, $R^3$, $R^4$ und $R^5$ die oben angegebene Bedeutung haben,

mit Verbindungen der Formel (IV)

$R^1$-X (IV)
in welcher
$R^1$ die oben angegebene Bedeutung hat, jedoch nicht Wasserstoff sein kann, und
X Chlor, Brom oder Iod bedeutet,
in Gegenwart von Säurebindern umsetzt.

10) Verwendung von 1-Cyclopropyl-6-fluor-1,4-dihydro-4-oxo-7-(alkyl-1-piperazinyl)-3-chinolincarbonsäuren der Formel

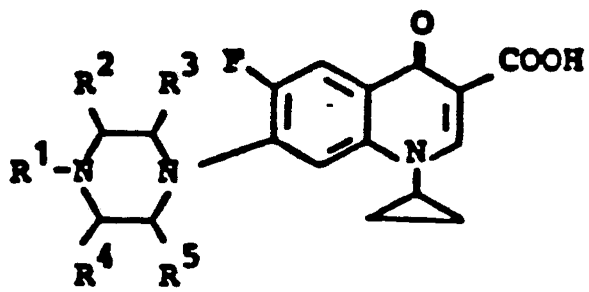

(I)

in der
$R^1$ für Wasserstoff oder gegebenenfalls durch Hydroxy substituiertes Alkyl mit 1 bis 12 Kohlenstoffatomen steht,
$R^2$, $R^3$, $R^4$ und $R^5$ gleich oder verschieden sein können und für Wasserstoff oder Alkyl mit 1 bis 4 Kohlenstoffatomen stehen, wobei mindestens einer der Reste $R^2$ bis $R^5$ Alkyl bedeutet,
und deren pharmazeutisch verwendbaren Säureadditions-, Alkali- und Erdalkalisalzen und Hydraten zur Herstellung von Arzneimitteln.

**Claims**

1) 1-Cyclopropyl-6-fluoro-1,4-dihydro-4-oxo-7-(alkyl-1-pipcrazinyl)-3-quinoline-carboxylic acids of the formula (I)

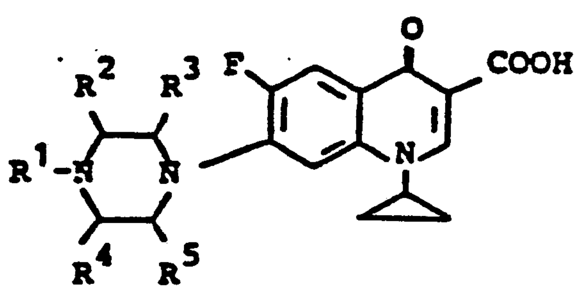

(I)

in which
$R^1$ represents hydrogen or alkyl which is optionally substituted by hydroxyl and has 1 to 12 carbon atoms,
$R^2$, $R^3$, $R^4$ and $R^5$ can be identical or different and represent hydrogen or alkyl having 1 to 4 carbon atoms, at least one of the radicals $R^2$ to $R^5$ denoting alkyl,
and their pharmaceutically utilisable acid addition, alkali metal and alkaline earth metal salts and hydrates.

2) 1-Cyclopropyl-6-fluoro-1,4-dihydro-4-oxo-7-(alkyl-1-piperazinyl)-3-quinoline-carboxylic acids of the formula (I)

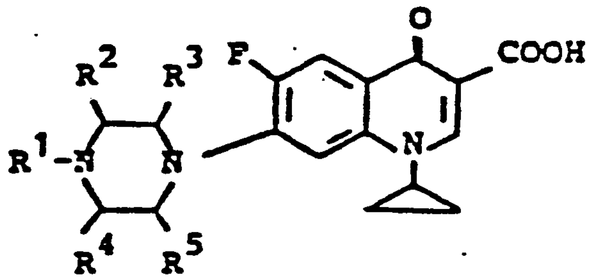

(I)

in which

$R^1$ represents hydrogen or alkyl which is optionally substituted by hydroxyl and has 1 to 4 carbon atoms,

$R^2$, $R^3$, $R^4$ and $R^5$ can be identical or different and represent hydrogen or alkyl having 1 to 2 carbon atoms, at least one of the radicals $R^2$ to $R^5$ denoting alkyl,

and their pharmaceutically utilisable acid addition, alkali metal and alkaline earth metal salts and hydrates.

3) 1-Cyclopropyl-6-fluoro-1,4-dihydro-4-oxo-7-(3-methyl-1-piperazinyl)-3-quinoline-carboxylic acid.

4) 1-Cyclopropyl-6-fluoro-1,4-dihydro-4-oxo-7-(3-ethyl-1-pipcrazinyl)-3-quinoline-carboxylic acid.

5) 1-Cyclopropyl)-6-fluoro-1,4-dihydro-4-oxo-7-(3,5-dimethyl-1-piperazinyl)-3-quinoline-carboxylic acid.

6) 1-Cyclopropyl-6-fluoro-1,4-dihydro-4-oxo-7-(4-ethyl-3-methyl-1-piperazinyl)-3-quinoline-carboxylic acid.

7) 1-Cyclopropyl-6-fluoro-1,4-dihydro-4-oxo-7-(4-ethyl-3,5-dimethyl-1-piperazinyl)-3-quinoline-carboxylic acid.

8) 1-Cyclopropyl-6-fluoro-1,4-dihydro-4-oxo-7-(alkyl-1-piperazinyl)-3-quinoline-carboxylic acids of the formula (I)

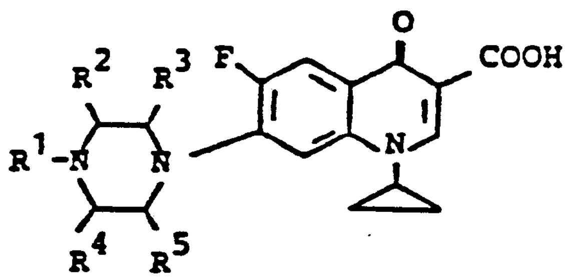

(I)

$R^1$ represents hydrogen or alkyl which is optionally substituted by hydroxyl and has 1 to 12 carbon atoms,

$R^2$, $R^3$, $R^4$ and $R^5$ can be identical or different and represent hydrogen or alkyl having 1 to 4 carbon atoms, at least one of the radicals $R^2$ to $R^5$ denoting alkyl,

and their pharmaccutically utilisable acid addition, alkali metal and alkaline earth metal salts and hydrates for the treatment of bacterial diseases.

9) Process for the preparation of 1-cyclopropyl-6-fluoro-1,4-dihydro-4-oxo-7-(alkyl-1-piperazinyl)-3-quinoline-carboxylic acids of the formula (1) in Claim 1, characterised in that either

a) 7-chloro-1-cyclopropyl-6-fluoro-1,4-dihydro-4-oxo-3-quinoline-carboxylic acids of the formula (II)

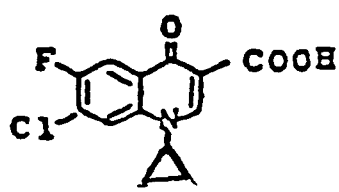

(II)

are reacted, in the presence of acid-binding agent, with piperazine derivatives of the formula (III)

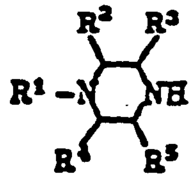

(III)

in which
$R^1$, $R^2$, $R^3$, $R^4$ and $R^5$ have the meanings indicated above,
or
b) compounds of the formula (IA) (= 1, $R^1$ = H)

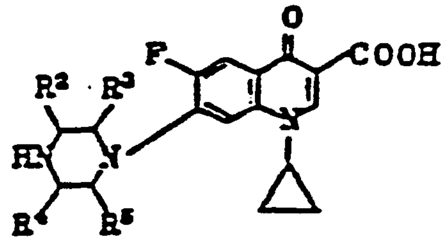

(IA)

in which
$R^2$, $R^3$, $R^4$ and $R^5$ have the meaning indicated above,
are reacted, in the presence of acid-binding agents, with compounds of the formula (IV)
$R^1$-X (IV)
in which
$R^1$ has the meaning indicated above, but cannot be hydrogen, and
X denotes chlorine, bromine or iodine.

10) Use of 1-cyclopropyl-6-fluoro-1,4-dihydro-4-oxo-7-(alkyl-1-piperazinyl)-3-quinoline-carboxylic acids of the formula (I)

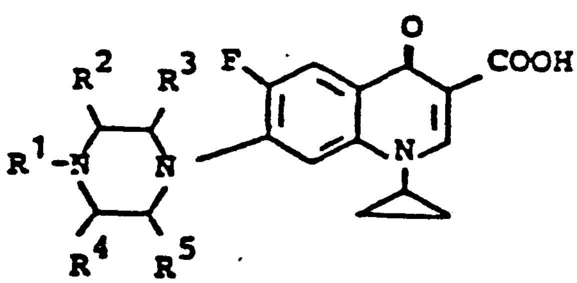

(I)

in which
$R^1$ represents hydrogen or alkyl which is optionally substituted by hydroxyl and has 1 to 12 carbon atoms,
$R^2$, $R^3$, $R^4$ and $R^5$ can be identical or different and represent hydrogen or alkyl having 1 to C carbon atoms, at least ont of the radicals $R^2$ to $R^5$ denoting alkyl,
and their pharmaceutically utilisable acid addition, alkali metal and alkaline earth metal salts and hydrates, for the production of medicaments.

**Revendications**

1. Acides 1-cyclopropyl-6-fluoro-1,4-dihydro-4-oxo-7-(alkyl-1-pipérazinyl)-3-quinoléine-carboxyliques de formule I

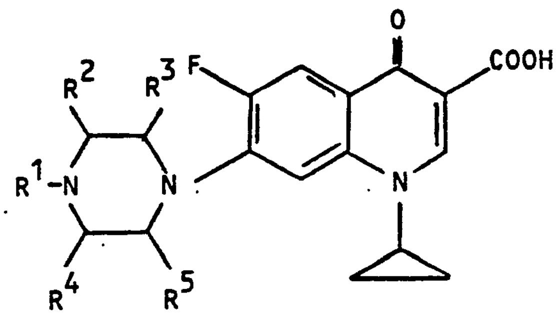

(I)

dans laquelle
$R^1$ représente l'hydrogène ou un groupe alkyle en $C_1$-$C_{12}$ éventuellement substitué par des groupes hydroxy,
$R^2$, $R^3$, $R^4$ et $R^5$, qui peuvent avoir des significations identiques ou différentes, représentent chacun l'hydrogène ou un groupe alkyle en $C_1$-$C_4$, l'un au moins les symboles $R^2$ à $R^5$ représentant un groupe alkyle,
et leurs sels formés par addition avec des acides, sels alcalins et sels alcalino-terreux et hydrates acceptables pour l'usage pharmaceutique.

2. Acides 1-cyclopropyl-6-fluoro-1,4-dihydro-4-oxo-7-(alkyl-1-pipérazinyl)-3-quinoléine-carboxyliques de formule I

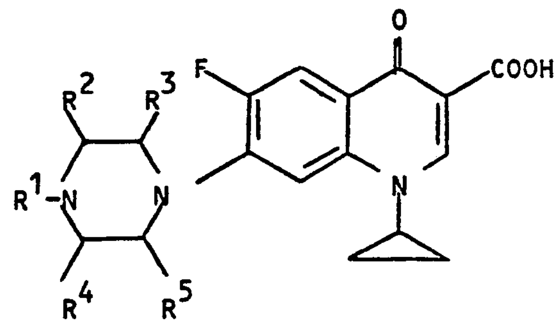

(I)

dans laquelle
$R^1$ représente l'hydrogène ou un groupe alkyle en $C_1$-$C_4$ éventuellement substitué par des groupes hydroxy,
$R^2$, $R^3$, $R^4$ et $R^5$, qui peuvent avoir des significations identiques ou différentes, représentent chacun l'hydrogène ou un groupe alkyle en $C_1$-$C_2$, l'un au moins des symboles $R^2$ à $R^5$ représentant un groupe alkyle,
et leurs sels formés par addition avec des acides, sels alcalins et alcalino-terreux et hydrates acceptables pour l'usage pharmaceutique.

3. L'acide 1-cyclopropyl-6-fluoro-1,4-dihydro-4-oxo-7-(3-méthyl-1-pipérazinyl)-3-quinoléine-carboxylique.

4. L'acide 1-cyclopropyl-6-fluoro-1,4-dihydro-4-oxo-7-(3-éthyl-1-pipérazinyl)-3-quinoléine-carboxylique.

5. L'acide 1-cyclopropyl-6-fluoro-1,4-dihydro-4-oxo-7-(3,5-diméthyl-1-pipérazinyl)-3-quinoléine-carboxylique.

6. L'acide 1-cyclopropyl-6-fluoro-1,4-dihydro-4-oxo-7-(4-éthyl-3-méthyl-1-pipérazinyl)-3-quinoléine-carboxylique.

7. L'acide 1-cyclopropyl-6-fluoro-1,4-dihydro-4-oxo-7-(4-éthyl-3,5-diméthyl-1-pipérazinyl)-3-quinoléine-carboxylique.

8. Acides 1-cyclopropyl-6-fluoro-1,4-dihydro-4-oxo-7-(alkyl-1-pipérazinyl)-3-quinoléine-carboxyliques de formule I

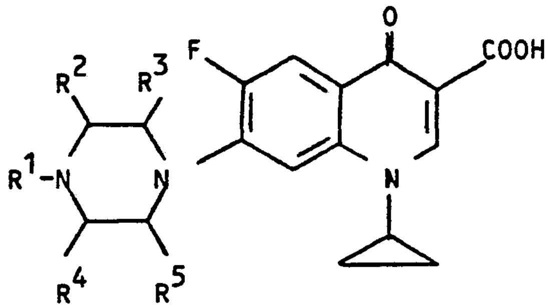

(I)

$R^1$ représente l'hydrogène ou un groupe alkyle en $C_1$-$C_{12}$ éventuellement substitué par des groupes hydroxy,

$R^2$, $R^3$, $R^4$ et $R^5$, qui peuvent avoir des significations identiques ou différentes, représentent l'hydrogène ou un groupe alkyle en $C_1$-$C_4$, l'un au moins des symboles $R^2$ à $R^5$ représentant un groupe alkyle,

et leurs sels formés par addition avec des acides, sels alcalins et alcalino-terreux et hydrates acceptables pour l'usage pharmaceutique, pour le traitement des maladies bactériennes.

9. Procédé de préparation des acides 1-cyclopropyl-6-fluoro-1,4-dihydro-4-oxo-7-(alkyl-1-pipérazinyl)-3-quinoléine-carboxylique de formule I de la revendication 1, caractérisé en ce que :

a) on fait réagir des acides 7-chloro-1-cyclopropyl-6-fluoro-1,4-dihydro-4-oxo-3-quinoléine-carboxyliques de formule II

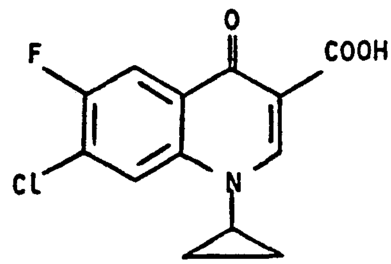

(II)

avec des dérivés de la pipérazine de formule III

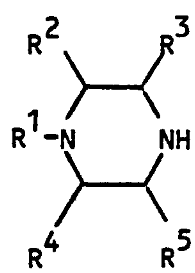

(III)

dans laquelle

$R^1$, $R^2$, $R^3$, $R^4$ et $R^5$ ont les significations indiquées ci-dessus, en présence d'agents fixant les acides, ou bien

b) on fait réagir des composés de formule IA (= I, $R^1$ = H)

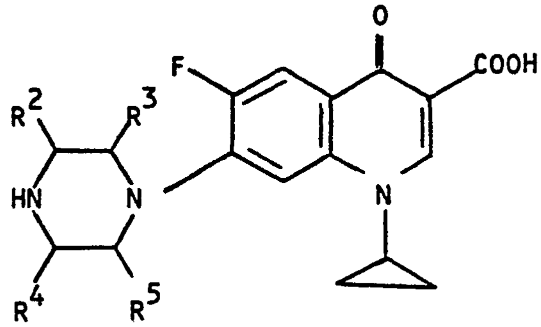

(IA)

dans laquelle

$R^2$, $R^3$, $R^4$ et $R^5$ ont les significations indiquées ci-dessus, avec des composés de formule IV

$R^1$-X (IV)

dans laquelle

$R^1$ a les significations indiquées ci-dessus, mais ne peut représenter l'hydrogène, et
X représente le chlore, le brome ou l'iode,
en présence d'agents fixant les acides.

10. Utilisation des acides 1-cyclopropyl-6-fluoro-1,4-dihydro-4-oxo-7-(alkyl-1-pipérazinyl)-3-quinoléine-carboxylique de formule I

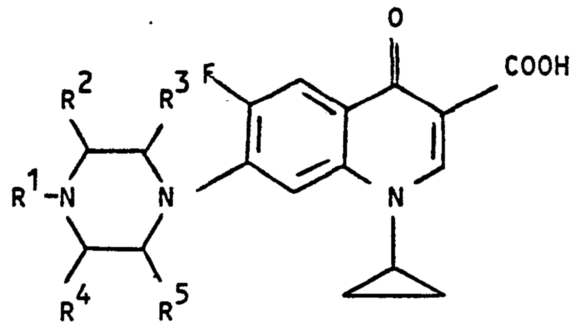

(I)

dans laquelle
$R^1$ représente l'hydrogène ou un groupe alkyle en $C_1$-$C_{12}$ éventuellement substitué par des groupes hydroxy,
$R^2$, $R^3$, $R^4$ et $R^5$, qui peuvent avoir des significations identiques ou différentes, représentent l'hydrogène ou un groupe alkyle en $C_1$-$C_4$, l'un au moins des symboles $R^2$ à $R^5$ représentant un groupe alkyle,
et de leurs sels formés par addition avec des acides, sels alcalins et alcalino-terreux et hydrates acceptables pour l'usage pharmaceutique, pour la préparation de médicaments.